(19) **Europäisches Patentamt**
**European Patent Office**
**Office européen des brevets**

(11) **EP 3 266 470 A1**

(12) **EUROPEAN PATENT APPLICATION**

(43) Date of publication:
**10.01.2018 Bulletin 2018/02**

(51) Int Cl.:
**A61K 51/00** (2006.01)     **A61B 6/00** (2006.01)
**A61P 35/00** (2006.01)

(21) Application number: **16178280.0**

(22) Date of filing: **06.07.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
- **Fyzikální ústav AV CR, v.v.i.**
  **25241 Dolni Brezany (CZ)**
- **Istituto Nazionale Di Fisica Nucleare (INFN)**
  **00044 Frascati RM (IT)**

(72) Inventors:
- **Giuffrida, Lorenzo**
  **25241 Dolni Brezany (CZ)**
- **Daniele, Margarone**
  **25241 Dolni Brezany (CZ)**
- **Korn, Georg**
  **25241 Dolni Brezany (CZ)**
- **Cirrone, Pablo**
  **95123 Catania (IT)**
- **Antonino, Picciotto**
  **38122 Trento (IT)**

Remarks:
Claim 18 is deemed to be abandoned due to non-payment of the claims fee (Rule 45(3) EPC).

(54) **DEVICE AND METHOD FOR IMAGING AND ENHANCED PROTON-THERAPY TREATMENT USING NUCLEAR REACTIONS**

(57)     The present invention provides a device, a system and a method for cancer therapy based on proton boron nuclear fusion reaction and simultaneous prompt gamma-ray imaging. The invention is based on the use of an incoming energetic proton beam interacting with a mixture of $^{11}B$ and $^{10}B$ isotopes to achieve simultaneity of the clinical irradiation and imaging, as well as to enhance the biological dose released into the cancer cells. The device is specifically adapted to performing such treatment.

Fig. 2

**Description**

**Technical Field**

The present invention discloses a device and a method for cancer treatment and simultaneous real-time imaging mainly based on nuclear reactions triggered by energetic protons produced in any accelerator. The invention further discloses a stand-alone diagnostic system suitable for characteristic gamma-rays performing medical imaging based on these nuclear reactions.

**Background Art**

Charged energetic particles are routinely used in medicine, in particular, cancer therapy. This type of treatment refers to hadron-therapy which is becoming one of the most important medical procedures to treat tumors instead of traditional radiotherapy treatments. However, further improvements can be carried out not only in terms of overall cost of a hadron-therapy center but also in terms of improvement of the treatment quality based on the tumor type and its location in the human body.

Although several candidates for this treatment technique in terms of ions radiation therapy have been considered, at the moment mainly protons and carbon ions have achieved satisfactory clinical results and are routinely used for cancer treatment. The main advantage of hadron-therapy compared to traditional radiotherapy is the fact that charged particles release most of their energy in a few millimeters close to the end of their penetration range (the so-called Bragg peak region). This important characteristic allows for critically damaging only tumor cells without dangerous interactions with healthy tissues in the human body surrounding the tumor region. Moreover, a clear enhancement of the Relative Biological Effectiveness (RBE) has been demonstrated in the case of carbon ions with respect to protons, resulting in a more efficient treatment. On the other hand, by using carbon ions issues connected with nuclear fragmentation can arise, thus leading to unwanted dose deposition beyond the Bragg peak. Moreover, nuclear fragmentation also causes an uncertainty on the biological dose ultimately associated to the cancer tissues.

Recently alternative techniques for cancer therapy are being considered in order to enhance the efficiency of the treatment. For instance, the nuclear reaction involving the interaction of thermal neutrons and the $^{10}B$ nuclei is already used in medicine for the cancer treatment in the so-called Boron Neutron Capture Therapy (BNCT). By using a syringe, a solution containing $^{10}B$ is injected in the human body surrounding the tumorregion.
The interaction of the neutron beam with $^{10}B$ nuclei triggers the following nuclear reaction:

$$^{10}B + n_{th} \rightarrow [^{11}B]^* \rightarrow \alpha + {}^{7}Li + 2.3 \text{ MeV} \tag{1}$$

where the final product is an energetic alpha-particle. These alpha-particles produced are mainly localized in the tumor region due to their short propagation length, leading to a more efficient interaction with the tumor cells.

However, unlike the classical hadron-therapy where the charged particle beam delivers a dose according to the Bragg-peak curve, in the BNCT the neutron beam is slowly attenuated in the human body, thus healthy tissues lying in the neutron beam injection path are unavoidably exposed to high neutron doses before the neutron beam hits the boron enriched tumor region and undergoes the above described nuclear reaction (1). BNCT requires a so-called epithermal neutron beam which requires special devices for its generation which up to now have very limited accessibility.

The $^{11}B(p, \alpha)2\alpha$ nuclear-fusion reaction was disclosed for first time in document M. L. E. OLIPHANT AND L. RUTHERFORD, et al. Experiments on the Transmutation of Elements by Protons. Proc. R. Soc. 1933, vol. 141, no.259. In this document, an energetic proton beam interacting with $^{11}B$ nuclei can trigger the following nuclear reaction

$$^{11}B + p \rightarrow 3\alpha + 8.7 \text{ MeV} \tag{2}$$

Theoretical calculations, confirmed by experimental measurements with standard accelerators have shown that the main channel of this reaction occurs with protons with energies around 600-700 keV. The result of such a main reaction channel is the generation of three alpha-particles with energies between 2.5 MeV and 5.5 MeV, with a maximum at 4.5 MeV.

Document W. M. NEVINS AND R. SWAIN. The Thermonuclear Fusion Rate Coefficient for p - 11 B Reactions. Nucl. Fusion. 2000, vol.40, p.865 discloses a secondary channel which is also present and generates alpha-particles in the 6-10 MeV energy range.

Document N. ROSTOKER, M.W. BINDERBAUER, AND H. J. MONKHORST. Colliding Beam Fusion Reactor.

Science. 1997, no.278, p.1419 discloses the main advantage of such a reaction which is totally clean with the meaning of no neutron generation. The document particularly refers to the possibility to build an ultraclean nuclear-fusion reactor, where the wording ultraclean means neglecting the number of erased neutrons.

Document A. PICCIOTTO, D. MARGARONE, A. VELYHAN, P. BELLUTTI, J. KRASA, A. SZYDLOWSKY, G. BERTUCCIO, Y. SHI, A. MANGIONE, J. PROKUPEK, A. MALINOWSKA, E. KROUSKY, J. ULLSCHMIED, L. LASKA, M. KUCHARIK and G. KORN. Boron-Proton Nuclear-Fusion Enhancement Induced in Boron-Doped Silicon Targets by Low-Contrast Pulsed Laser, Phys. Rev. X 4, 031030 (2014) discloses the possibility to trigger such a nuclear reaction by using a high power pulsed laser interacting with solid B-enriched target.

Documents KR 101568938 B and DO-KUN YOON, JOO-YOUNG JUNG, AND TAE SUK SUH. Application of proton boron fusion reaction to radiation therapy: A Monte Carlo simulation study. Applied Physics Letters. 2014, vol.105, p.223507 disclose a scheme where the alpha-particles generated by the interaction of a proton beam with only $^{11}$B nuclei has been proposed as Proton Boron Fusion Therapy (PBFT).

## Summary of invention

The invention disclosed in this document provides a device and a method for prompt gamma-ray imaging and simultaneous proton therapy using nuclear reactions, wherein an irradiated object comprises a composition comprising mixture of $^{11}$B and $^{10}$B.

The interaction of protons with $^{10}$B nuclei, comprised in the composition, wherein the composition is held in irradiated object, triggers additional nuclear reactions generating characteristic prompt gamma-rays which can be used for real-time monitoring, particularly 2D or 3D medical imaging, of the treatment, and potentially for a dynamic treatment with a feedback control based on the real-time dose measurement, particularly dose delivery system.

The characteristic prompt gamma-ray peaks due to the interaction of the $^{10}$B nuclei with the energetic protons are present at 429 keV, 718 keV and at 1430 keV. The peak at 429 keV is ascribed to the $^{10}$B $(p,\alpha)$ $^{7}$Be nuclear reaction. The peak at 718 keV is mainly ascribed to the inelastic scattering of the $^{10}$B ($^{10}$B* $(p,p')$$^{10}$B) but it can also come out of the $^{10}$B $(p,n)$ $^{10}$C reaction and the consequent $^{10}$C $\beta$+ decay in the $^{10}$B*.

The $^{11}$B nuclei, interacting with protons, can trigger a nuclear reaction ($^{11}$B $(p,2n)$ $^{10}$C) $\beta$ + decaying in $^{10}$B* emitting a gamma-ray at 718 keV, but the cross-section of this reaction is very low compared to the other cross-section of reactions with $^{10}$B nuclei.

The peak at 1430 keV is due to the $^{10}$B$(p,p')$$^{10}$B* nuclear reaction and it happens when the $^{10}$B* decay from the level at 2.15 MeV to the level 718 keV. There are other characteristic gamma-ray peaks, due to the elastic scattering with protons with $^{10}$B (3 MeV, 5.91 MeV, 6.02 MeV and 6.5 MeV) and with $^{11}$B (4.82 MeV).

In the present invention, it is essential that both $^{11}$B and $^{10}$B are present in the isotope mixture. The $^{11}$B nuclei are important mainly for triggering the p-11B nuclear reaction, while the $^{10}$B nuclei are important mainly for the prompt gamma-emission, which occurs simultaneously and thus allows carrying out a dynamic treatment based on real-time imaging.

The ratio of $^{11}$B and $^{10}$B in the mixture may vary based on the type and location of the irradiated object. In particular the irradiated object is a tumor.

For superficial tumors it is convenient to use isotope mixture comprising 10%-50% of $^{10}$B and 90%-50% of $^{11}$B in order to maximize the production of the gamma prompt peaks that are exclusive for the p-10B reaction.

For deeper and radiation-resistant tumors, starting for example from a depth of 10 cm of soft tissue, as the gamma attenuation in the tissues will reduce the total amount of detectable gammas, it is more convenient to use the ratio of 0.1%-20% of $^{10}$B and 99.9%-80% of $^{11}$B to produce an additional gamma peak at 4.82 MeV, which is generated in an elastic scattering reaction. More preferably, the ratio will be 1%-20% of $^{10}$B and 99%-80% of $^{11}$B, or 3%-20% of $^{10}$B and 97%-80% of $^{11}$B, or 5%-20% of $^{10}$B and 95%-80% of $^{11}$B.

For radiation-resistant tumors at intermediate tumor depth (e.g. melanoma or glioma) it is more convenient to use the ratio of 0.1%-30% of $^{10}$B and 99.9%-70% of $^{11}$B, while for non-radiation-resistant tumors at intermediate tumor depth (e.g. medulloblastoma or sarcoma) it is more convenient to use the ratio of 15%-40% of $^{10}$B and 85%-60% of $^{11}$B.

In various situations and as per the consideration of the clinician, all the above-described ratios may be combined. In case of personalized treatment, it will mostly be preferred to use other than the naturally occurring ratio of $^{11}$B and $^{10}$B.

The isotopes $^{11}$B and $^{10}$B may be present in the mixture in the form of any compound used or usable for the purposes of radiation therapy, with the corresponding ratios of $^{11}$B and $^{10}$B isotopes. Suitable compounds are well known to a person skilled in the art. Such compounds may include, e.g., boronophenylalanine, sodium borocaptate, dodecaborate cluster lipids and cholesterol derivatives, $Na_2B_{10}H_{10}$, cholesteryl ester mimics, boronated DNA metallo-intercalators, transferrin-polyethylene glycol liposomes, boron-containing unnatural amino acids, dodecahydro-closo-dodecaborate clusters, carboranyl nucleosides, carboranyl porphyrins, carboranyl porphyrazines, boronated cyclic peptides, boronated antibodies, boron-containing nanoparticles, boron carbide particles, boron containing immunoliposomes and liposomes, boronated EGF and anti-EGFR and VEGFR MoAbs and boron nitride nanotubes.

A device for simultaneous treatment and/or calibration, and imaging comprises a source of energetic protons coming from an accelerator system, said accelerator system being preferably tunable in energy from 0.1 MeV to 400 MeV.

In a preferred embodiment, the energetic protons are generated by a standard accelerator.

In another preferred embodiment, the source of energetic protons is a laser-driven accelerator. One of the advantages of the laser-driven accelerator is its compactness.

The device further comprises at least one transport line for delivering the proton beam to at least one treatment station. The transport line ends with a tube which is responsible for collimation and/or direction of the proton beam and wherein the proton beam is focused to an irradiated object.

The treatment station further preferably comprises a positioning system. To this positing system, there are attached devices wherein the positioning system adjusts the spatial position of any of the attached devices according to a treatment session and/or data obtained from imaging, e.g. evaluated by computer during irradiation. The positioning system can also control the location of irradiated object, wherein the movement can be provided longitudinally, transversally or the devices attached to the positioning system can revolve around the irradiated object.

In certain embodiment, the irradiated object is a water phantom.

In another embodiment, the irradiated object can be at least one or a plurality of tumors located in a human or animal body.

The treatment station further comprises a system which provides real-time imaging. The system for real-time imaging comprises at least two gamma-ray detectors, particularly, sensitive for given gamma-ray peaks characterized by its energy. The system for real-time imaging can optionally comprise a computer and/or computational system and/or any means for evaluation and/or storing of data obtained from gamma-ray detectors. The energy of given gamma-ray peak is preferably 429 keV, 718 keV and 1430 keV. Other gamma-ray peaks are with energy of 3 MeV, 5.91 MeV, 6.02 MeV and 6.5 MeV for elastic hadronic scattering from $^{10}$B nuclei and the peak at 4.82 MeV coming from elastic hadronic scattering from $^{11}$B nuclei. The gamma-ray detectors are connected to the above mentioned evaluation system capable for real-time imaging. Moreover the gamma-ray detectors can be devices attached to the positioning system. The layout and/or spatial position of the gamma ray detectors for imaging is adapted according to the position of irradiated object so that, the detectors are able to provide at least 2D medical scan.

As an advantage, in another preferred embodiment, the number of gamma-ray detectors is to be able to provide a 3D medical scan.

The gamma-ray detectors are covered by collimators to shield the radiation background, particularly, the undesired gamma rays.

In preferred embodiment, the collimators are from Pb material.

Another device or plurality of devices, optionally attached to the positioning system, and connected to the computational system is at least one or a plurality of gamma-ray detector for spectra analysis.

In preferred embodiment, the detection of gamma-rays for imaging purpose is provided by conventional gamma-ray detectors, particularly, silicon or germanium gamma-ray detectors sensitive for 429 keV, 718 keV, 1430 keV, 3 MeV, 4.82 MeV, 5.91 MeV, 6.02 MeV and 6.5 MeV characteristic peak from the reaction coming from the interaction of protons with B isotopes.

In another preferred embodiment the system for real-time imaging is a single Photon Emission Tomography (SPECT) detector.

In preferred embodiment, the position of at least two detectors for imaging is orthogonal with respect to each other.

Further devices attached to the positioning system optionally are at least one tube capable for collimation and/or direction of proton beam and at least one of the above mentioned radiation detector for whole spectra and/or background monitoring.

Moreover, the positioning system comprises a bed where the irradiated object is fixed during the treatment. The devices connected to the positioning system are able to change their spatial position, in the above mentioned degree of freedom, according to the irradiated target, wherein in certain example, the target is a tumor located in a patient. The positioning system comprises a system for fixing the object or patient, and/or avoiding unwanted and/or dangerous movements during the treatment.

In the preferred embodiment, the bed is located in the center of the positioning system so that, the attached devices is able to provide their functions around the irradiated object.

Further, the treatment station comprises the electronic part, which serves to in-situ position adjustment and automatic movement depending on the position of an irradiated object that can be controlled remotely by a screen including all adjustable parameters of the treatment.

In the preferred embodiment, the positioning system is a circular shape. The circular shape provides effective movement of devices which are attached to this positioning system. The irradiated object is located in center of the positioning system. The positioning system comprises plurality of radiation detectors attached to the positioning system and plurality gamma-ray detectors used for detection of given gamma-rays. Signals from the gamma-ray detector are processed and/or evaluated and/or stored by the computation system for the real-time imaging. The computation system images dose deposition in the irradiated object and provides 2D, optionally 3D, image of the irradiated body. The radiation detector can move transversally and/or longitudinally and also can revolve around the irradiated object depending on the evaluation from the real-time imaging computation system. The radiation detectors are preferably gamma-ray detectors which are arranged around the irradiated object such that the sensitive part of the detection device is targeted to the irradiated object. The gamma-ray detectors have a pinhole (with a variable diameter of the hole) in front of the sensitive part to avoid all the background. The devices comprised in system for the real-time imaging can move transversally and longitudinally and also can revolve around the irradiated object depending on evaluation from the real-time imaging computation system and they move independently on each other and/or additional devices attached to the positioning system. The imaging system is further covered by a pinhole system, with a different hole aperture, in order to select only the right gamma-ray peak component by avoiding all background radiation.

The location of all the devices attached to the positioning system is changeable and depending on the tumor location in the object. The position of the tube for the incoming proton beam is chosen in order to be as close as possible to the tumor region.

In the preferred embodiment the gamma-ray devices are placed at 90° with respect to the interaction of the proton beam to the irradiated object. The gamma-ray devices have the sensitive part of the detector in front of the irradiated object. In the preferred embodiment the system for the imaging must be placed, directed to the irradiated object, parallel to the proton beam.

Further, the irradiated object comprises a reagent agent comprising at least $^{10}$B and $^{11}$B in calculated ratio. The calculation is provided by dedicated software. The $^{10}$B serves for medical imaging; $^{11}$B serves for producing reaction channel according to eq. (2).

In certain embodiments, the composition is a water solution and itcomprises further elements which are helpful for biological transport of the mixture $^{10}$B/$^{11}$B into the tumor. The elements are preferably Na, H, S, C in different relative concentrations, but generally, created compound with B (composed of $^{10}$B and $^{11}$B). The ratio of $^{10}$B/$^{11}$B is given by calculation with dedicated software and depends on the tumor type and location. The concentration of boron mixture, with given ratio of $^{10}$B and $^{11}$B, is ranging from 10 ppm to $10^4$ ppm in the solution.

In another embodiment, the composition is a saline solution with boron concentration ranging from 10 ppm to $10^4$ ppm, wherein the ratio of $^{10}$B/$^{11}$B is given by the calculation according to the dedicated software.

In another embodiment, the composition of boron mixture is prepared in form of a pill or pills.

In one embodiment, the relative ratio of $^{10}$B vs. $^{11}$B is 20% vs. 80%, the natural isotope of boron.

In the preferred embodiment, the irradiated object is a phantom, preferably a water phantom, with density close to the density of the tissue, whereas into the phantom is situated a target including the composition with prescribed concentration of the calculated mixture of $^{10}$B/$^{11}$B.

In another preferred embodiment, the irradiated object is a patient, wherein the composition of boron is absorbed by the cancer cell regions in the patient.

Invention also discloses a system suitable only for imaging and/or calibration based on prompt gamma-rays coming from nuclear reaction(s) with $^{10}$B and/or $^{11}$B. Said system further comprises a source of energetic protons forming a proton beam, at least one diagnostic station (104), at least one transport line (103) for delivering the proton beam to the at least one treatment station (104), said transport line (104) having an output tube (400) adapted for collimation and/or direction and/or focusing of the proton beam, and a system for real-time imaging comprising at least two gamma-ray detectors (603,604), and a composition comprising a $^{10}$B and $^{11}$B isotope.

The system comprises a step of introducing the composition containing $^{10}$B and/or $^{11}$B (20%-100% of $^{10}$B and 80%-0% of $^{11}$B) in the range from 10 to $10^4$ ppm, into a target region which is in demand to be imaged. The target region is situated in the irradiated object.

The irradiated object can be a water phantom. In this case the calibration is used for imaging the part of the phantom simulating the cancer region, having a density equivalent to the human body and containing the composition doped with boron exactly in the same position and depth where the cancer is located in the patient.

The invention can also used for imaging the tumor region situated in the human body of the patient.

The irradiated object comprising target region with composition is situated into the positioning system. The diagnostics devices, preferably at least two gamma-ray detectors for real time imaging, sensitive for 429 keV, 718 keV, 1430 keV, 3 MeV, 4.82 MeV, 5.91 MeV, 6.02 MeV and 6.5 MeV characteristic peak, and at least two radiation detectors for whole spectra monitoring and proton source tube are set to default position. Protons with energy preferably in range 0.1 MeV to 400 MeV are accelerated by standard accelerator or by laser-driven accelerator; proton beam is guided by the transport line to the at least one treatment station. The protons are targeted, through the tube attached to the positioning system, to the irradiated body. The protons irradiate the target region, wherein the protons interact with the $^{10}$B and $^{11}$B nuclei in the target region. The reaction starts to produce prompt gamma-rays, which are detected by gamma-ray detectors. The radiation background can be simultaneously monitored by another radiation detector. The information on gamma-rays intensity is processed by the system for real-time imaging according to the dedicated software. The software can be commercially available software for treatment planning and dose deposition planning. Based on the real-time imaging, the proton tube position is adjusted by the positioning system to the position most suitable for irradiation. The gamma-ray detector for imaging or a plurality of gamma-ray detectors for imaging and radiation detector or a plurality of radiation detectors for whole spectra and/or gamma background are adjusted according to the evaluation based on the real-time imaging system.

In preferred embodiment the composition contains only $^{10}$B nuclei in the range from 10 to $10^4$ ppm, into a target region and the gamma ray detectors are sensitive for 429 keV, 718 keV, 1430 keV, 3 MeV, 5.91 MeV, 6.02 MeV and 6.5 MeV characteristic peak.

This invention also provides a method for imaging and enhanced proton-therapy treatment using nuclear reactions. Prior the treatment a calibration test is performed by using a phantom, having a density equivalent to the human body and containing the composition doped with boron exactly in the same position and depth where the cancer is located in the patient. The calibration is useful because it allows evaluating the real components of the human body (B dose and concentration on the tumor region, the flux of protons sent to the patient) for the treatment. Moreover, based on the calibration test, the relative concentration $^{10}$B/$^{11}$B is determined. For example, if the tumor is located much too deep or is covered by too much fat, the relative concentration of $^{10}$B will increase to improve the imaging effect.

The method comprises the steps of introducing the composition with prescribed concentration and calculated relative ratio of $^{10}$B and $^{11}$B into a target region. The target region is situated in the irradiated object, preferably the water phantom. The position and shape of the target region is located in the same position and depth as a tumor in a patient. The irradiated object comprising target region with composition is situated into the positioning system. The diagnostics devices, preferably at least two gamma-ray detectors for real time imaging and at least two radiation detectors for whole spectra monitoring and proton source tube are set to default position. Protons with energy preferably in range 0.1 MeV to 400 MeV are accelerated by standard accelerator or by laser-driven accelerator; proton beam is guided by the transport line to the at least one treatment station. The protons are targeted, through the tube attached to the positioning system, to the irradiated body. The protons irradiate the target region, wherein the protons interact with both the $^{11}$B and $^{10}$B nuclei in the target region. The reaction starts to produce prompt gamma-rays, which are detected by gamma-ray detectors. The radiation background can be simultaneously monitored by another radiation detector. The information on gamma-rays intensity is processed by the system for real-time imaging according to the dedicated software. The software can be commercially available software for treatment planning and dose deposition planning. Based on the real-time imaging, the proton tube position is adjusted by the positioning system to the position most suitable for irradiated. The gamma-ray detector for imaging or a plurality of gamma-ray detectors for imaging and radiation detector or a plurality of radiation detectors for whole spectra and/or gamma background are adjusted according to the evaluation based on the real-time imaging system.

In another example, the method comprises the steps of introducing the solution with prescribed boron concentration and calculated relative ratio of $^{10}$B and $^{11}$B into a tumor in the patient. The patient with tumor and with solution, wherein the solution is absorbed by the tumor, is situated into the positioning system. The diagnostics devices, preferably at least two gamma-ray detectors for imaging and at least two radiation detectors for spectra monitoring and/or background monitoring and the proton source tube are set to a position established by a calibration test on water phantom. A proton beam, tunable in energy from 0.1 to 400 MeV, irradiates the tumor, wherein the protons interact with both the $^{11}$B and $^{10}$B nuclei surrounding the tumor. In special cases, lower energy ions in the range from 100 keV to tens MeV could also be used for very shallow located tumors or tumor cells. The interaction between protons and boron nuclei triggers the p-$^{11}$B nuclear reaction generating alpha-particles that are localized mainly in the cancer region inside the cancer cells since the alpha particle propagation range is comparable to the cell size. The great advantage of these alpha-particles is that, because of their proximity to the cancer cells, they do not release energy in healthy tissues but release their dose only in cancer cells, enhancing considerably the damage in the cancer region. Additionally, this type of treatment utilizes the benefits of both protons and heavier particles (carbon ions for instance) in cancer therapy. In fact, protons are here besides their typical Bragg peak behavior mainly used as bullets to trigger the nuclear reaction, and because of the well-known Bragg peak they are able to penetrate up to the tumor region minimizing the damage (dose release) to the healthy tissue along the beam path. On the other hand, the alpha-particles generated by the nuclear reaction behave as typical heavy ions in cancer therapy, i.e. they have a higher LET (linear energy transfer) and generate more efficient damages in the single cell, thus enhancing the RBE. However, since the alpha particles are generated directly in the tumor region, it is possible to avoid the side effects typical of heavy ion therapy, such as nuclear fragmentation along the beam propagation in the healthy tissues and damages in the region behind the cancer, which is the main problem in the case of carbon therapy. Furthermore, due to the higher RBE only in the tumor region, our invention allows enhancing the biological dose released into the cancer cells and minimizing it in the surrounding healthy tissues by using the same physical dose compared to conventional proton-therapy.

These and other aspects of the present invention will be readily apparent to those skilled in the art in view of the following drawings and detailed description. The summary and the following detailed description are not considered restrictive of the invention as defined in the appended claims and serve only to provide examples and explanations of the invention.

**Advantages of the invention**

Compared to conventional proton-therapy our invention allows for the increase in the degrees of freedom connected to the possibility to vary the proton beam parameters, for instance the proton flux and energy, depending on the best treatment and planning for the specific patient (tumor type, tumor location, maximum allowed dose in the specific part of the human body). In fact, for given proton beam parameters, the concentration of the solution, and especially the relative ratio of isotopes 11B and 10B in the solution, can vary and be optimized in order to have a sort of personalized treatment for each patient.
This applies also according to clinical situation which can further be combined with other methods to individualize treatments. This seems to be a necessary development due to the stated fact that tumors show a very high heterogeneity.
For this reason the presence of an optimized mixture of at least $^{11}$B and $^{10}$B is mandatory for the invention hereby described. The $^{11}$B nuclei are important mainly for triggering the p-11 B nuclear reaction, while the $^{10}$B nuclei are important mainly for the prompt gamma-emission, which can occur simultaneously thus allowing to carry out a dynamic treatment based on a real-time imaging.

**Brief description of drawings**

Fig. **1** is a schematic diagram of a proton accelerator - treatment station units in a multi-station center for cancer treatment.
Fig. **2** is a schematic diagram of the positioning system for the diagnostic and the cancer treatment.
Fig. **3** represents gamma-ray spectra emitted in the case of the $^{10}$B, $^{11}$B and $^{nat}$B (80% of 11 B and 20% of $^{10}$B), respectively in a), b) and c).
Fig. **4** shows 718 keV gamma-ray emissions from the tumor region at different depth vs different B isotope concentrations.
Fig. **5** depicts the relative dose released by protons within the human body in the case of a reference water phantom (dashed line) and in the case of the presence of $^{11}$B at 1% concentration (black line) and 100% concentration (gray dashed line) within the Bragg peak region (a).
Fig. **6**: alpha-particle a) and proton b) yield within the irradiated sample.
Fig. **7**: scheme showing the difference between the conventional proton - therapy (a) and the treatment with alpha-particles (b) is the proton beam (energy from 0.1 to 400 MeV), is the human body, are the cancer cells, is the Bragg peak, are the B nuclei and are the alpha-particles.
Fig. **8**: represents the basic flowchart showing an overview of an example method for automated treatment.

**Detailed description**

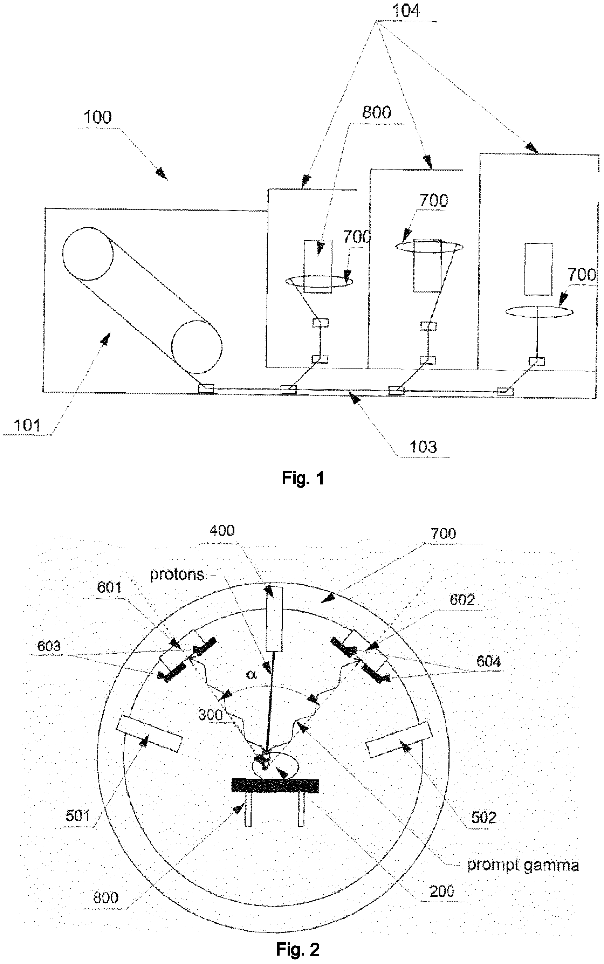

Fig. 1 shows a multi-station device **100** with three treatment stations **104** accompanied by a positioning system **700**. The multi-station device **100** comprises a standard accelerator **101,** which accelerates protons. The protons are subsequently guided through a transport line **103** to the positioning system **700**. The positioning system **700** is circular shape and further comprises a tube **400** attached to this positioning system **700**. The transport line **103** can lead to more than one treatment station **104** and, thus, more than one irradiation session can be provided at the same time.
Fig. 2 shows a medical device for simultaneous treatment and imaging. The device comprises a tube **400** attached to the positioning system wherein the irradiated object **200** is situated on the bed **800** during the irradiation. The bed is situated in the middle of the ring shape positioning system. The detectors for imaging **601,602** are covered by pinholes **603,604** to shield gamma-rays which do not serve for online medical imaging. The angle $\alpha$ between two gamma detectors for imaging is in preferred embodiment 90°. The device further comprises detectors **501,502** for radiation monitoring. The position of gamma detectors **501,502,601,602** can change during irradiation, although the angle between detector **601** and **602** must be sufficient for medical imaging.
In one example, the proton beam pulse with energy 60 MeV is directed into the irradiated object contained in water including a solution with isotopes of $^{10}$B nuclei, $^{11}$B nuclei and $^{nat}$B nuclei. The characteristic gamma-rays coming out of the irradiated object was up to 1.5 MeV during a potential cancer treatment. For this example, a Ge detector **601,602** with resolution less than 1 keV is used. A particular focus is given to the following gamma-ray peaks: 429 keV, 718 keV and 1430 keV. The Ge gamma-ray cylindrical-shell detector **601,602** is used in the simulations in order to maximize the signal coming out of the irradiated sample.

In another example, the proton beam pulse with energy of 60 MeV is directed into the irradiated object contained in water including a solution with isotope of $^{10}B$ nuclei and $^{11}B$ nuclei. The characteristic gamma-rays coming out of the irradiated object was from 3 MeV up to 6 MeV during a potential cancer treatment. A particular focus is given to the following gamma-ray peaks: 3 MeV, 5.91 MeV, 6.02 MeV, 6.5 MeV (for $^{10}B$ nuclei) and 4.82 MeV (for $^{11}B$ nuclei).

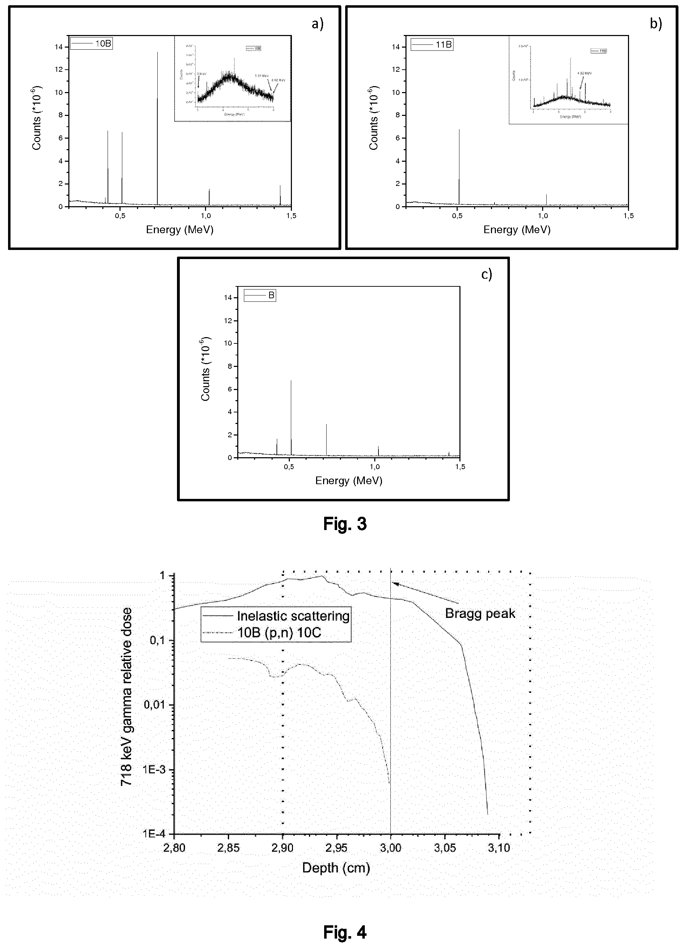

In another example, another object is irradiated by the proton beam with energy of 60 MeV: the water reference sample and the water samples doped with 1 % of $^{nat}B$, with 1% of $^{10}B$ and with 1% of $^{11}B$ in the Bragg peak region. The final results are shown in Fig.3 a) b) and c) respectively for $^{10}B$, $^{11}B$ and $^{nat}B$.

The first piece of important evidence is clear from the reference sample test (water phantom) where the above mentioned gamma-ray peak is not present. Only a peak at 511 keV is dominant and is ascribed to the annihilation radiation of the electron-positron pair. In the case of the $^{10}B$ (shown in Fig.3a) there are different important prompt gamma peaks: 718 keV (most important and intense), 429 keV and 1430 keV. Other peaks (with lower intensity) are 3 MeV, 5.91 MeV, 6.02 MeV, 6.5 MeV.

Completely different simulation outputs are reported in Fig. 3b showing the gamma-ray spectrum emitted from the sample containing only $^{11}B$. Here only the presence of the peak at 718 keV is registered in the energy range up to 1.5 MeV, however the peak intensity at 718 keV is much lower than the 718 keV peak obtained in the case of the sample containing only $^{10}B$. Another peak at 4.82 MeV is registered in the energy range from 3 MeV up to 6 MeV. Two important evidences come out of the comparison between the gamma spectrum produced by the $^{10}B$ doped sample and the one produced by the $^{11}B$ doped sample. In the latter case the gamma peak at 429 keV is no present, and the peak at 718 keV is very low. This is a crucial point since for the proposed simultaneous imaging and treatment technique the presence of both boron isotopes in an optimized relative percentage (depending on the patient disease) becomes mandatory.

Fig. 3c shows the results obtained with the $^{nat}B$ doped sample. These results are in agreement with what discussed above and the gamma ray peaks at 429 and 718 keV are shown with different intensities. More details are reported in Table I where the peak intensity at different energies, normalized to the peak at 718 keV from the $^{10}B$, is compared for the different cases. Various information can be extracted from that comparison. First of all the peak at 718 keV from $^{11}B$ is more than 30 times lower than the same which comes out from the $^{10}B$. The peaks at 429 keV and 1430 keV are present only with $^{10}B$ (and natural B). In the case of $^{11}B$ these peaks disappear. Once again this means that the presence of $^{10}B$ is fundamental for the diagnostic of the characteristic gamma-rays.

Another important point is to understand the origin of the above mentioned main characteristic gamma ray peaks and also to use them for imaging. In the case of the $^{10}B$ doped sample (but also for the $^{nat}B$ doped one which contains 20% of $^{10}B$) the 718 keV gamma-rays emission is mainly due to the inelastic scattering of the $^{10}B$ nuclei with protons: $^{10}B(p,p'y)^{10}B$. This is an aneutronic nuclear reaction having a non-negligible cross-section for energies up to 10 MeV. The following nuclear reactions are also possible: $^{10}B(p,n)^{10}C$ and $^{10}C$ decay $\beta+$ going to the $^{10}B^*$ exited state and emitting a gamma-ray at 718 keV. However, only the inelastic reaction leads to a prompt gamma peak, while the other reactions do not generate prompt gamma radiation. The interaction of the protons with $^{10}B$ nuclei can be also responsible of another nuclear reaction: $^{10}B(p,a)^7Be$. In such reaction the final result is a prompt gamma-ray emission at 429 keV.

Thus, the above identified nuclear reactions can explain the origin of the peaks obtained in the examples. More details are shown in Fig. 4 where the rates of the gamma peaks corresponding to the different reactions are compared. The relative reaction rate of the inelastic scattering is compared to the rate of the $^{10}B(p,n)^{10}C$ nuclear reaction. In the area of interest (within the Bragg peak region) the relative rate of the $^{1O}B(p,n)^{10}C$ reaction is less than the 10% compared to the inelastic scattering one. This clearly explains that the main origin of the 718 keV is ascribable to the $^{10}B(p,p'\gamma)^{10}B$ inelastic scattering.

The same plot also confirms that the presence of $^{10}B$ is mandatory to perform imaging since the contribution of the 718 keV gamma peak produced in the $^{11}B$ doped sample is much lower (only 5%) than the one produced in the $^{10}B$ doped sample and, moreover, the gamma peak produced in the $^{11}B$ doped sample is not prompt since it can be produced only through the $^{11}B(p,2n)^{10}C$ nuclear reaction.

In order to have a personalized treatment the crucial point is the choice of the right concentrations of the different compounds in the solution. The gamma emission is strongly connected to the depth of the tumor location in the human body, which strongly influences the relative concentration of $^{10}B$ compared to $^{11}B$.

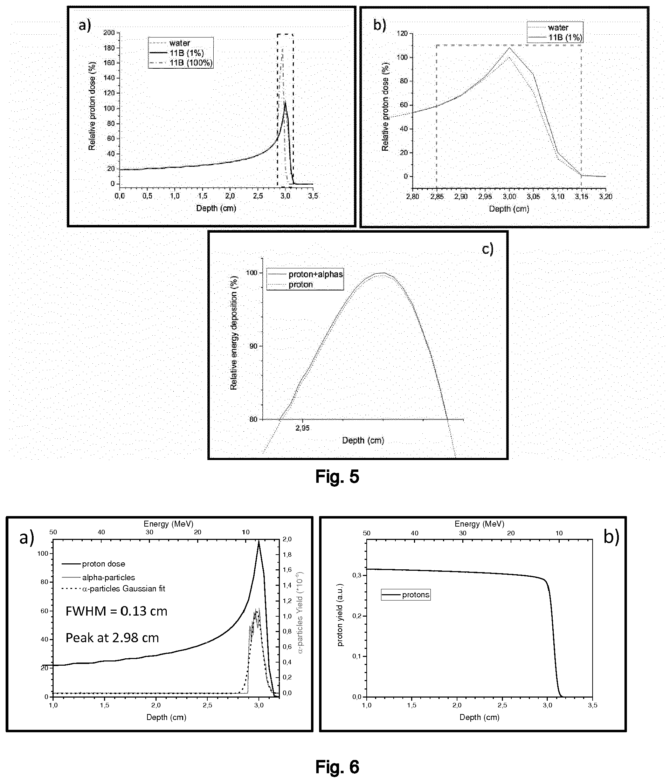

In a different example a water object is irradiated with a 60 MeV proton beam. The dose released by protons in that reference object (in the grey dotted line), giving a peak at 3 cm depth, is compared with the released dose of a sample having the same size but with a solution containing a small concentration of $^{11}B$ (1% in water, in black line) in the Bragg peak region. Fig. 5a and Fig.5b report this comparison. The calculated values are normalized here considering the water object result as the reference case (100% at the peak). A small enhancement (less than 10%) of the total physical dose released within the Bragg peak region in the case of the sample $^{11}B$ (1%) is here evident. The position of the Bragg peak in both cases is practically the same. This result shows that the small enhancement in the physical dose observed in the case of $^{11}B$ (1%) is not ascribable to the alpha particles produced in the p-$^{11}B$ nuclear reaction but mainly due to the presence of the $^{11}B$ atoms, which changes the composition and the density of the water phantom. Fig.5 a shows also an unrealistic case (grey dotted line) where the $^{11}B$ within the Bragg peak region is pure (100%). A large enhancement of the Bragg peak compared to the reference object case is shown (from 100% to 180%), thus clearly demonstrating that this change is ascribable to the different density of the irradiated sample. Fig.5c shows the relative energy deposition for protons (in black line) and for protons + alpha-particles (in the dotted black line) for the $^{11}$B (1%) sample. The comparison shows that the deposited energy is coming from the protons since the two curves are practically the same (only 0.3% of the total contribution is ascribable to the alpha-particles).

In a different example a water object containing 1% of $^{11}$B within the Bragg peak region, is irradiated with 60 MeV of protons. In Fig. 6a the alpha-particle yield (black line) is shown in comparison to the dose absorbed in the whole sample (grey line). A Gaussian fit of the alpha-particle distribution is carried out (dashed black line). The low FHWM of the curve (1.3 mm) leads to the conclusion that the alpha-particles are is confined in a very well localized region, thus allowing to define very precisely the position where they release the dose during the treatment. Another important point is that the peak of the alpha-particles is placed approximately in the same position where the protons release their maximum dose (i.e. around the Bragg peak). Thus, the exact knowledge of the Bragg peak position and of the position of the peak of maximum alpha-particle dose release can be used to enhance the efficacy of the treatment. Fig.6b shows the number of protons passing through the irradiated sample. This plot is in agreement with the one in Fig.5a, showing that the amount of protons starts to decrease in the Bragg peak region, falling down to zero after a few mm. It is important to note that the relative amount of alpha-particles (Fig. 6a) compared to protons (Fig. 6b) is much lower (around 5 orders of magnitude). This again means that the physical dose enhancement close to the Bragg peak (around 10%) is mainly due to the protons and only in minor amount to the alpha-particles generated in the nuclear fusion reaction.

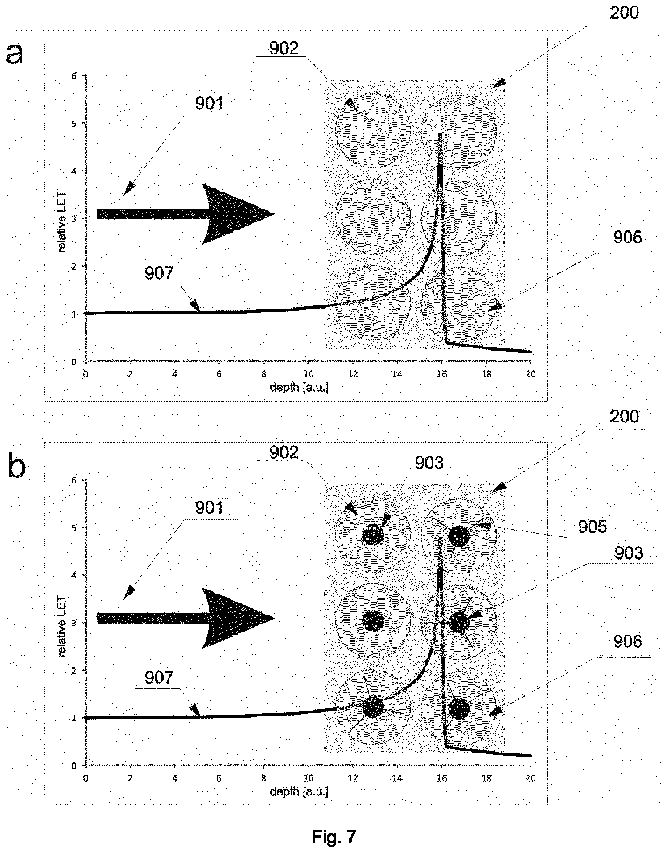

Figure 7 represents the proton beam **901** irradiating the patient's body **200,** wherein the body includes healthy cells **902** and tumor cells **906**. Both, healthy cells **902** and tumor cells **906** comprise boron mixture **903** depending on previously performed calculation. Due to proton beam **901** set-up based on calculation and testing on water phantom, the proton beam **901** has lost the major part of its original energy in the region, represented by Bragg peak **907,** in the region, where the tumor cells **906** are located. Due to the reaction (2) the tumor cells **906** are fully destroyed by alpha particles **905** with energy of few MeV (2-5 MeV), having a range of penetration in the tumor cells **906** of less than 20 micrometers. Since the typical size of cancer cells is around 20-30 microns, this confirms that most of the alpha-particles release their dose inside the cells, thus enhancing the possibility to destroy only the tumor cells and to spare the healthy tissues in the patient's body **200.** Thus, although the alpha-particles generated in the nuclear fusion reaction is very low compared to the total number of protons (low physical dose enhancement), the RBE is largely enhanced due to the location of the alpha-particles inside the tumor cells.

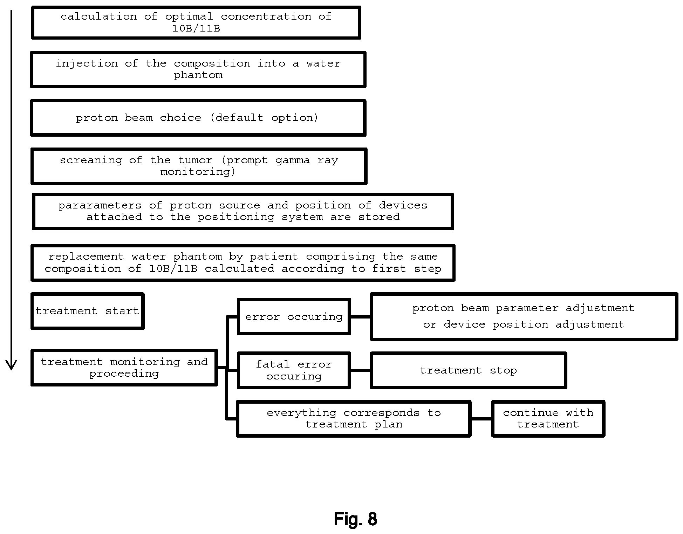

The method of personalized treatment, represented by Fig. 8, comprises the step of calculation of the boron mixture to be injected to the object and also the calibration of the proton beam parameters for the irradiation. A screening of the tumor is carried out in order to localize with high precision the tumor in the object. Software is able to calculate the right parameters for the treatment (i.e. proton energy and boron concentration). According to this measurement, the solution containing boron is injected into a water phantom, having the same shape and density as the patient. After this calibration test, if everything corresponds to the demanded released dose according to treatment planning, the same parameters are used for the real treatment. During the treatment the safety system, included in the treatment station, checks in real-time all information coming from the gamma-ray spectra and also of the imaging system. If something goes wrong it can change different parameters to correct the error. If the error is impossible to be solved in real-time, the system stops automatically the treatment for the necessary adjustments.

In one example, for superficial tumors it is most convenient to increase the $^{10}$B with respect to the $^{11}$B concentration (10%-50% of $^{10}$B and 90%-50% of $^{11}$B) in order to maximize the production of the gamma prompt peaks that are exclusive of the p+$^{10}$B reaction. The presence of these exclusive peaks, will improve the quality of the beam imaging process. In this case the software calculates the relative concentration $^{10}$B/$^{11}$B and after the calibration, if everything corresponds to the demand dose release, the same solution is injected into the object, previously fixed on the bed of the treatment station.

In another example, for deeper and radiation-resistant tumors, starting from a depth of 10 cm of soft tissue, as the gamma attenuation in the tissues will reduce the total amount of detectable gammas, it is more convenient to increase the percentage of $^{11}$B with respect to the $^{10}$B (0.1%-20% of $^{10}$B and 99.9%-80% of $^{11}$B) to produce an additional gamma peak at 4.82 MeV, which is generated in an elastic scattering reaction. This will affect the enhancement of the tumor treatment but also maximize the efficiency and the quality of the imaging. In fact, although the cross-section of such elastic scattering reaction is relatively low, the high energy gamma-ray peak (4.82 MeV) will suffer a lower attenuation in the tissues compared to the lower energy gamma-ray peaks.

In another example if the enhancement of the proton biological effectiveness is not requested for the specific treatment (e.g. not radio-resistant tumors), the content of $^{10}$B can be increased in order to maximize the presence of the exclusive gamma peaks (429 keV, 718 keV, 1430 keV, 3 MeV, 5.91 MeV, 6.02 MeV and 6.5 MeV), thus to increase the efficiency and quality of the imaging. In this case the Boron injection is performed only for imaging purposes (100% of $^{10}$B).

**Industrial applicability**

The present invention can find application particularly in medical field and generally, in any field where on-line imaging and simultaneous energy release is used, e.g. energy distribution monitoring. The present invention can be employed in the detection of gamma rays generated during interaction of B nuclei (with different concentration of isotopes $^{10}$B and $^{11}$B) with protons for real-time imaging technique. The present invention can be also employed for the combination of cancer treatment using the p-$^{11}$B nuclear reaction and the on-line diagnostic.

**Claims**

1.   A composition comprising $^{11}$B and $^{10}$B isotopes for use in a diagnostic method or simultaneous diagnostic and treatment of tumors by energetic protons and proton boron fusion and simultaneous prompt gamma-ray imaging.

2.   The composition for use according to claim 1, comprising 10%-50% of $^{10}$B and 90%-50% of $^{11}$B.

3.   The composition for use according to claim 1, comprising 0.1%-20% of $^{11}$B and 99.9%-80% of $^{11}$B.

4.   The composition according to anyone of preceding claims, wherein the $^{10}$B or $^{11}$B is comprised in at least one of the following compositions: boronophenylalanine, sodium borocaptate, dodecaborate cluster lipids and cholesterol derivatives, $Na_2B_{10}H_{10}$, cholesteryl ester mimics, boronated DNA metallo-intercalators, transferrin-polyethylene glycol liposomes, boron-containing unnatural amino acids, dodecahydro-closo-dodecaborate clusters, carboranyl nucleosides, carboranyl porphyrins, carboranyl porphyrazines, boronated cyclic peptides, boronated antibodies, boron-containing nanoparticles, boron carbide particles, boron containing immunoliposomes and liposomes, boronated EGF and anti-EGFR and VEGFR MoAbs and boron nitride nanotubes.

5.   A device for simultaneous prompt gamma-ray imaging and treatment and/or calibration, comprising a source of energetic protons forming a proton beam, at least one treatment station (104), at least one transport line (103) for delivering the proton beam to the at least one treatment station **(104),** said transport line **(104)** having an output tube **(400)** adapted for collimation and/or direction and/or focusing of the proton beam, and a system for real-time imaging comprising at least two gamma-ray detectors **(603,604).**

6.   The device according to claim 5, wherein said source of energetic protons is a laser driven accelerator of protons.

7.   The device according to claim 5 or 6, wherein the source of energetic protons comprises an accelerator system **(101),** said accelerator system being preferably tunable in energy from 0.1 MeV to 400 MeV.

8.   The device according to claim 5 to 7, wherein the treatment station **(104)** comprises a positioning system **(700)** for adjusting the spatial position of any of the components of the device.

9.   The device according to any one of claims 5 to 8, wherein the gamma-ray detectors **(603,604)** are adapted to detect one or more peaks selected from peaks having the energy 429 keV, 718 keV and 1430 keV, and preferably also 3 MeV, 4.82 MeV, 5.91 MeV, 6.02 MeV and 6.5 MeV.

10.  The device according to any one of claims 5 to 9, further comprising at least one gamma-ray detector **(501 or 502)** for spectral analysis.

11.  The device according to any one of claims 5 to 10, further comprising a bed **(800)** for fixing an irradiated object **(200)** or a patient, preferably said bed **(800)** is located in the center of the positioning system **(700).**

12.  The device according to any one of claims 5 to 11, further comprising at least one control unit for controlling the proton beam energy and targeting and/or positioning of individual components of the device and/or treatment regimen, preferably the control unit comprises a feedback loop for controlling the operation of the device based on the prompt gamma-ray imaging outputs.

13.  A system for simultaneous prompt gamma-ray imaging and treatment and/or calibration, comprising the device according to any one of claims 5 to 12, and a composition according to any one of claims 1 to 4.

**14.** The system according to claim 13, wherein the mixture comprises 0.1%-50% of $^{10}$B and 99.9%-50% of $^{11}$B.

**15.** The system according to claim 13 or 14, wherein the concentration of boron composition comprising $^{10}$B/$^{11}$B is ranging from 10 to $10^4$ ppm.

**16.** A system for prompt gamma-ray imaging and/or calibration, comprising a source of energetic protons forming a proton beam, at least one diagnostic station (104), at least one transport line (103) for delivering the proton beam to the at least one treatment station (104), said transport line (104) having an output tube (400) adapted for collimation and/or direction and/or focusing of the proton beam, and a system for real-time imaging comprising at least two gamma-ray detectors (603,604), and a composition comprising a $^{10}$B and/or $^{11}$B isotope, wherein said composition comprising 10%-50% of $^{10}$B and 90%-50% of $^{11}$B.

**17.** A method for calibration of the device according to any one of claims 5 to 12 or the system according to any one of claims 13 to 15 or the system according to claim 16, said method comprising the steps of

- introducing a composition comprising a mixture of $^{10}$B and $^{11}$B into a target region within a water phantom,
- targeting an energetic proton beam to the target region, thereby initiating interaction of the protons with both the $^{11}$B and $^{10}$B nuclei in the target region
- detecting prompt gamma-rays produced by the interaction of the protons with boron nuclei,
- using data obtained from said detection of prompt gamma-rays for adjusting the proton beam source to a position most suitable for irradiation.

**18.** A method for irradiation of tumors using the device according to any one of claims 5 to 12 or the system according to any one of claims 13 to 15 or the system according to claim 16, said method comprising the steps of

- introducing a composition comprising a mixture of $^{10}$B and $^{11}$B into a target region which is the tumor,
- targeting an energetic proton beam to the target region, thereby initiating interaction of the protons with both the $^{11}$B and $^{10}$B nuclei in the target region,
- detecting prompt gamma-rays produced by the interaction of the protons with boron nuclei,
- using data obtained from said detection of prompt gamma-rays for controlling and/or adjusting the treatment regimen.

**Fig. 1**

**Fig. 2**

**Fig. 3**

**Fig. 4**

**Fig. 5**

**Fig. 6**

**Fig. 7**

calculation of optimal concentration of 10B/11B

injection of the composition into a water phantom

proton beam choice (default option)

screaning of the tumor (prompt gamma ray monitoring)

parameters of proton source and position of devices attached to the positioning system are stored

replacement water phantom by patient comprising the same composition of 10B/11B calculated according to first step

treatment start

treatment monitoring and proceeding

error occuring → proton beam parameter adjustment or device position adjustment

fatal error occuring → treatment stop

everything corresponds to treatment plan → continue with treatment

**Fig. 8**

Europäisches
Patentamt

European
Patent Office

Office européen
des brevets

## EUROPEAN SEARCH REPORT

Application Number

EP 16 17 8280

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHIN HAN-BACK ET AL: "Prompt gamma ray imaging for verification of proton boron fusion therapy: A Monte Carlo study", PHYSICA MEDICA, ACTA MEDICA EDIZIONI E CONGRESSI, ROME, IT, vol. 32, no. 10, 24 May 2016 (2016-05-24), pages 1271-1275, XP029786785, ISSN: 1120-1797, DOI: 10.1016/J.EJMP.2016.05.053 | 1-3 | INV. A61K51/00 A61B6/00 A61P35/00 |
| Y | * the whole document * | 4 | |
| X | ROLF F BARTH ET AL: "Current status of boron neutron capture therapy of high grade gliomas and recurrent head and neck cancer", RADIATION ONCOLOGY, BIOMED CENTRAL LTD, LO, vol. 7, no. 1, 29 August 2012 (2012-08-29), page 146, XP021116850, ISSN: 1748-717X, DOI: 10.1186/1748-717X-7-146 | 1-4 | |
| Y | * the whole document and in particular the abstract and Table 1 * | 4 | TECHNICAL FIELDS SEARCHED (IPC) A61K |

~~The present search report has been drawn up for all claims~~

| Place of search | Date of completion of the search | Examiner |
|---|---|---|
| The Hague | 6 April 2017 | Birikaki, Lemonia |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

Application Number

EP 16 17 8280

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the
requirements of unity of invention and relates to several inventions or groups of inventions, namely:

1. claims: 1-4

    A composition comprising 11B and 10B for use in a diagnostic
    method or simultaneous diagnostic and treatment of tumors by
    energetic protons and proton boron fusion and simultaneous
    prompt gamma-ray imaging.
    ---

2. claims: 5-17

    A device according to claim 5, a system according to claims
    13 or 16 and a method for calibration according to claim 17
    ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101568938 B

### Non-patent literature cited in the description

- **M. L. E. OLIPHANT ; L. RUTHERFORD et al.** Experiments on the Transmutation of Elements by Protons. *Proc. R. Soc.,* 1933, vol. 141 (259
- **W. M. NEVINS ; R. SWAIN.** The Thermonuclear Fusion Rate Coefficient for p - 11 B Reactions. *Nucl. Fusion.,* 2000, vol. 40, 865
- **N. ROSTOKER ; M.W. BINDERBAUER ; H. J. MONKHORST.** Colliding Beam Fusion Reactor. *Science,* 1997, (278), 1419
- **A. PICCIOTTO ; D. MARGARONE ; A. VELYHAN ; P. BELLUTTI ; J. KRASA ; A. SZYDLOWSKY ; G. BERTUCCIO ; Y. SHI ; A. MANGIONE ; J. PROKU-PEK.** Boron-Proton Nuclear-Fusion Enhancement Induced in Boron-Doped Silicon Targets by Low-Contrast Pulsed Laser. *Phys. Rev.,* 2014, vol. 4, 031030
- **DO-KUN YOON ; JOO-YOUNG JUNG ; TAE SUK SUH.** Application of proton boron fusion reaction to radiation therapy: A Monte Carlo simulation study. *Applied Physics Letters,* 2014, vol. 105, 223507